Europäisches Patentamt

European Patent Office

Office européen des brevets

(19)

(11) Numéro de publication : **0 566 448 A1**

# (12) DEMANDE DE BREVET EUROPEEN

(21) Numéro de dépôt : **93400851.7**

(51) Int. Cl.$^5$ : **C08J 3/20**, G01N 33/58

(22) Date de dépôt : **02.04.93**

(30) Priorité : **07.04.92 FR 9204217**

(43) Date de publication de la demande :
**20.10.93 Bulletin 93/42**

(84) Etats contractants désignés :
**DE ES FR GB IT**

(71) Demandeur : **PROLABO**
**12 rue Pelée**
**F-75526 Paris Cédex 11 (FR)**

(72) Inventeur : **Ricchiero, Frédéric**
**Le Domitien-B46, 23, rue Jacques Brive**
**F-34090 Montpellier (FR)**
Inventeur : **Richard, Jöel**
**Résidence Mermoz, 15, rue Marie Amélie**
**F-60500 Chantilly (FR)**
Inventeur : **Vaslin, Sopie**
**Parc des Bords de Marne, 53bis, Quai Louis**
**Ferber**
**F-94360 Bry/Sur/Marne (FR)**

(74) Mandataire : **Schrimpf, Robert et al**
**Cabinet Regimbeau 26, Avenue Kléber**
**F-75116 Paris (FR)**

(54) **Latex fluorescent possédant un seuil de détection en émission fluorescente très faible.**

(57)   La présente invention a pour objet un latex fluorescent dont les particules contiennent au moins un fluorochrome hydrophobe encapsulé caractérisé en ce qu'il possède un seuil de détection de fluorescence inférieur ou égal à $10^{-12}$ moles de particules par litre de latex et en ce que ledit fluorochrome hydrophobe encapsulé dans les particules est un polyaromatique condensé, un de ses dérivés substitués, la tétraphénylporphine ou un des complexes organométalliques de celle-ci.
   La présente invention se rapporte également à un procédé de préparation dudit latex et à ses applications.

EP 0 566 448 A1

La présente invention a pour objet un latex fluorescent doté d'un rendement quantique élevé, d'un seuil de détection en fluorescence très bas et dont les particules incorporent au moins un fluorochrome hydrophobe.

Les latex sont des dispersions aqueuses de particules de polymère, dont la taille est généralement comprise entre 0,1 microns et plusieurs microns. Compte-tenu de leur aspect particulaire, les latex offrent une grande surface spécifique qui est exploitée avantageusement dans un grand nombre d'applications et en particulier dans les industries de peinture, bandes magnétiques, enregistrements et en biologie.

En biologie et plus particulièrement dans le domaine de l'immunologie, des techniques de dosages classiques mettent en oeuvre des latex à titre de support de marqueurs de type enzymes ou radio-isotopes afin de permettre le dosage quantitatif des espèces présentes dans les milieux à analyser.

Cette technique de dosage par marquage moléculaire à l'aide d'isotope radioactif, est extrêmement précise, fiable et d'une grande sensibilité qualitative. Toutefois, elle présente plusieurs inconvénients liés à la dispersion des sources radio-actives, l'exposition prolongée du personnel, la variation temporelle de l'émission de la source liée à la demi-vie de l'élément et surtout à la nécessité en fin d'analyse d'isoler les réactifs libres des réactifs complexés. L'émission radioactive est en effet totalement insensible à l'environnement du marqueur.

Pour les raisons précitées, la technique d'analyse par fluorescence s'est imposée comme l'alternative la plus efficace pour le remplacement de ces marqueurs classiques.

Les avantages du marquage fluorescent, comparativement au marquage radio-actif, sont multiples. Le risque d'exposition radio-active est nul. Le marquage fluorescent présente une excellente stabilité dans le temps et surtout il fournit une réponse plus affinée en raison de la spécificité de l'émission de fluorescence à certains paramètres d'environnement. Son emploi ne nécessite donc pas la mise en oeuvre de séparations post-analyses.

La mise en oeuvre pratique de la fluorescence, dans les dosages immunologiques, reste donc simplement subordonnée à un exigence de sensibilité quantitative. Sa sensibilité doit être comparable à celle du marquage radio-actif ce qui implique de pouvoir travailler à des concentrations comprises entre $10^{-12}$ et $10^{-15}$ mole de particules par litre.

Actuellement, c'est précisément au niveau de sa sensibilité que se situe la limite d'utilisation de la fluorescence.

La fluorescence, issue du simple greffage moléculaire, présente une sensibilité insuffisante en vue d'une application pratique généralisée. Il est difficile de la détecter lorsque la concentration des entités présentes dans le milieu est inférieure à $10^{-9}$ mole/litre.

La présente invention a précisément pour objectif de pallier à cet inconvénient.

Elle propose une nouvelle gamme de latex fluorescents qui, de par le mode de greffage du fluorochrome, la nature chimique de celui-ci et son taux d'occupation au sein des particules de latex possède un rendement quantique élevé et un seuil de détection de fluorescence très bas.

En outre, par opposition au greffage fluorescent moléculaire, les latex selon l'invention présentent l'avantage d'être constitués de particules dont la surface externe demeure suffisamment libre pour permettre une adsorption et éventuellement le greffage ultérieur de molécules biologiques.

Plus précisément, la présente invention se rapporte à un latex fluorescent dont les particules contiennent au moins un fluorochrome hydrophobe encapsulé caractérisé en ce qu'il possède un seuil de détection de fluorescence inférieur ou égal à $10^{-12}$ moles de particules par litre de latex et en ce que ledit fluorochrome hydrophobe et encapsulé au sein des particules du latex est un polyaromatique condensé, un de ses dérivés substitués, la tétraphénylporphine et/ou un des complexes organométalliques de celle-ci.

Par le terme encapsulé, on entend le fait que les fluorochromes sont quasiment absents de la surface des particules. Ils sont pour l'essentiel concentrés au sein des particules de monomères de manière à laisser la surface externe de celles-ci disponible.

Les fluorochromes selon l'invention sont sélectionnés de manière à couvrir en émission les quatre grandes zones du spectre lumineux à savoir le bleu (400-500 nm), le vert (500-550 nm), le jaune (550-600 nm) et le rouge (600-750 nm).

Outre les propriétés spectrales requises, ces fluorochromes satisfont également aux exigences suivantes:

Ils sont hydrophobes de manière à favoriser leur insertion dans les particules de latex et s'affranchir de tout relargage ultérieur.

Ils présentent une affinité pour l'intérieur de la particule c'est à dire sont compatibles chimiquement avec le polymère constituant les particules de latex et, le cas échéant, avec les fonctions chimiques présentes sur ce polymère. Cette compatibilité joue un rôle crucial lors de la synthèse du latex fluorescent correspondant.

Enfin, compte-tenu de leur emploi en immunologie, ces fluorochromes sont stables chimiquement et photochimiquement.

Parmi les fluorochromes convenant à l'invention on peut plus particulièrement citer les 9,10 diphényl-anthracènes (9,10 DPA), 9-10 bis-phényl-éthynyl-anthracènes (9-10 BPEA), 1, 8 dichloro 9-10 bis-phényl-éthynyl-anthracènes (1,8 Cl$_2$-9-10.BPEA), 5-12 bis-phényl-éthynyl-naphtacènes (5-12 BPEN) 6, 13-bis (phényléthynyl)pentacènes, tétrabenzo(de,hi,op,st)pentacènes, leurs dérivés substitués, la tétraphénylporphine et la tétraphénylporphine de zinc.

De manière générale, les fluorochromes sont choisis en fonction de la finalité et des conditions d'utilisation des particules de latex.

Les 9,10 bis-phényl-éthynyl-anthracènes émettent entre 480 et 550 nm c'est à dire dans le vert. Ces molécules sont fortement émettrices et possèdent des rendements quantiques proche de l'unité. A titre de dérivés substitués du BPEA, émettant dans ces mêmes longueurs d'ondes, on peut en particulier citer le 2-éthyl-9,10-BPEA, le 1-chloro-9,10-BPEA, le 2-chloro-9,10-BPEA, le 1,4-diméthyl-9,10-BPEA, le 1-phényl-9,10-BPEA et le 1,4-diphényl-9,10-BPEA.

Les 5-12 bis-phényl-éthynyl-naphtacènes (5-12 BPEN) de même que le 1,8 dichloro 9,10 BPEA émettent entre 560 et 650nm.

Quant aux tétraphényl porphine, tétraphényl porphine de zinc, 6,13-bis (phényléthynyl)pentacène, tétrabenzo(de,hi,op,st)pentacène, ils émettent en lumière rouge.

Les fluorochromes précités sont avantageux à plusieurs titres.

Il s'agit de produits commerciaux et peu coûteux. Ils sont insolubles dans l'eau et sont chimiquement et photochimiquement stables. Compatibles avec les polymères de latex, ils conduisent, lors de leur incorporation au sein des particules de latex, à des marqueurs possédant une sensibilité inattendue et nettement plus performante que celles des marqueurs classiques.

Les particules de latex contenant lesdits fluorochromes sont classiquement constituées de polymères obtenus par polymérisation de monomères insaturés éthyléniquement. Il s'agit d'un homopolymère ou copolymère contenant des motifs dérivés de monomères vinylaromatiques, éthyléniques, d'acides ou d'esters alcanoïques ou éthyléniques, éventuellement fonctionnalisés.

Ce type de polymère est facilement accessible à tout homme de l'art et nous nous contenterons d'en citer quelques uns ci-après, à titre non limitatif de l'invention. Il peut s'agir de :

- monomères éthyléniques de type isoprène, 1,3 butadiène, chlorure de vinylidène, acrylonitrile,
- monomères vinylaromatiques comme le styrène, le bromostyrène, l'alphaméthylstyrène, l'éthylstyrène, le vinyltoluène, le chlorostyrène ou le chlorométhylstyrène ou le vinylnaphtalène,
- acides, esters ou anhydrides alcanoïques comme les acides acrylique, méthacrylique, acrylates et méthacrylates d'alkyle dont le groupe allkyle possède 3 à 10 atomes de carbone, hydroxyacrylates, les esters d'acides éthyléniques à 4 ou 5 atomes de carbone ainsi que,
- les copolymères de ces monomères entre eux tels que le divinylbenzène ou le diacrylate de 2,2-diméthyl-1,3-propylène et/ou d'autres monomères copolymérisables insolubles dans l'eau.

Les monomères peuvent porter des groupements anioniques ou cationiques de type sulfate, sulfonate, phosphonate ou ammonium quaternaire. Il peut également s'agir de groupements susceptibles de réagir, directement ou indirectement, avec des groupements fonctionnels de type amine par exemple, portés par des molécules biologiques comme les protéines et les enzymes. A titre représentatif de ces groupes fonctionnels, on peut citer les halogènes, les groupements carboxyles, amines, isocyanates, aziridines, aldéhydes, sulfonyles et les fonctions époxy.

Les monomères, plus particulièrement utilisés dans le cadre de la présente invention, appartiennent à la famille des arylènes et/ou des alkylènes. Il s'agit préférentiellement de composés vinylaromatiques tels que: styrène, alphaméthylstyrène, éthylstyrène, tertio-butylstyrène et vinyltoluène. De préférence, ces monomères sont substitués par un ou plusieurs groupements fonctionnels de type halogène, amine, alkoxy, carboxyle et/ou sulfonyle.

Ces monomères sont utilisés seuls ou en mélange entre eux en toute proportion, ou encore en mélange avec un autre monomère copolymérisable choisis parmi ceux précités.

Les particules de polymères peuvent être obtenues par la mise en oeuvre d'une quelconque technique de polymérisation comme la polymérisation en émulsion classique, en microémulsion ou le cas échéant par polymérisation en milieu organique. Ces techniques familières à l'homme de l'art ne seront pas rappelées ici.

Les particules, constituant le latex fluorescent selon l'invention, sont hydrophobes et possèdent de préférence une taille généralement comprise entre 0,01 micron et 20 microns et de préférence inférieure à 5 microns. Elles sont calibrées, monodispersées et présentes dans le latex à raison d'une quantité variant entre 0,2 à 50% en poids du poids total du latex.

La présente invention se rapporte également à un procédé de préparation des latex fluorescents.

Le marquage fluorescent est réalisé par insertion des fluorochromes sélectionnés au sein des particules, selon des techniques dites de surpolymérisation ou de gonflement au solvant.

Plus précisément, la présente invention se rapporte à un procédé pour préparer un latex fluorescent mettant en oeuvre une étape choisie parmi:

- l' introduction, en cours de polymérisation du ou des monomère(s) constituant les particules du latex, d'un fluorochrome en suspension dans une fraction du ou d'un des monomères et compatible avec l'eau,
- le mélange d'un fluorochrome hydrophobe et solubilisé dans un solvant non aqueux compatible avec l'eau, avec une dispersion aqueuse de particules de latex,

caractérisé en ce que ledit fluorochrome mis en oeuvre est choisi parmi un polyaromatique condensé, ses dérivés substitués, la tétraphénylporphine ou les complexes organométalliques de celle-ci et en ce que l'on récupère en fin de réaction un latex possèdant un seuil de détection de fluorescence inférieur à $10^{-12}$ moles de particules par litre de latex.

La technique de surpolymérisation consiste en une synthèse de type "core-shell". Une semence de latex est surpolymérisée par un monomère en présence d'initiateur et d'un tensio-actif. Le fluorochrome, en suspension dans une fraction de ce monomère, est introduit en fin de polymérisation de façon à obtenir des billes de latex, d'une granulométrie précise et resserrée dans lesquelles le fluorochrome est encapsulé à une distance plus ou moins grande du coeur des particules. Après polymérisation, le latex est "strippé" afin d'éliminer toute trace de monomères résiduels, puis purifié.

Selon la méthode de gonflement par solvant, les particules de latex, sous forme d'une dispersion aqueuse, sont mises en contact du fluorochrome hydrophobe solubilisé dans un solvant, non aqueux et capable de gonfler les dites particules. Cette absorption est de préférence réalisée en élevant la température. L'augmentation de température, associée à la présence de solvant, est telle qu'elle permet de se placer au delà de la température de transition vitreuse du polymère constitutif des particules. Il en résulte que les chaînes macromoléculaires de polymère acquièrent une mobilité suffisante pour permettre la pénétration du fluorochrome qui est très hydrophobe. La valeur de cette température est bien entendu fonction des caractéristiques du polymère constituant les particules et de l'affinité du fluorochrome pour celui-ci. De manière générale elle varie entre 30°C et 95°C.

L'addition de la solution de fluorochrome est régulée dans le temps afin d'éviter tout phénomène de précipitation du composé hydrophobe au sein de la solution aqueuse et également la formation d'agglomérats de particules de polymères.

Au bout d'un certain temps, le solvant organique est éliminé par distillation lente ce qui entraîne la précipitation du fluorochrome à l'intérieur des particules de latex. Les particules de latex fluorescents sont ensuite purifiées selon des techniques de purification classiques.

Les solvants non miscibles à l'eau sont de préférence choisis parmi l'acétone, le chloroforme, le dichlorométhane, le cyclohexanone, le xylène et le toluène.

Les particules de latex fluorescents, obtenues à l'issue de l'une ou l'autre de ces deux méthodes, sont ensuite caractérisées en terme de taux d'occupation en fluorochromes, de fluorescence résiduelle et de sensibilité de détection de l'émission de fluorescence.

Le taux d'occupation maximal des particules de latex en fluorochromes hydrophobes est bien entendu fonction de la nature du fluorochrome, de la technique d'insertion mise en oeuvre, de la nature du polymère constituant les particules et de la taille de ces particules. Toutefois, ce taux possède une valeur telle que le seuil de détection en fluorescence est d'au plus de $10^{-12}$, de préférence entre $10^{-12}$ et $10^{-18}$ moles de particules par litre de latex. Ce taux d'occupation peut donc varier considérablement et atteindre des valeurs de plusieurs millions de molécules de fluorochromes par particule de latex.

A titre indicatif, pour une particule d'un diamètre de 0,5 $\mu$m, ce taux varie de 10.000 à 400.000, de préférence entre 60.000 et 350.000, molécules de fluorochromes par particule de latex.

L'incorporation des fluorochromes à l'intérieur des particules, selon l'une ou l'autre des deux techniques précitées, est avantageuse à deux titres:

Elle permet de s'affranchir complètement de tout phénomène de relargage de l'agent fluorochrome au cours du dosage. Aucun phénomène de fluorescence dite résiduelle n'est noté dans le surnageant. Il s'en suit une fiabilité accrue de la technique de dosage.

La surface externe des particules de latex demeure disponible pour des couplages chimiques ou biologiques.

Selon un mode de réalisation particulier de l'invention, le latex fluorescent est en outre fonctionnalisé par au moins une espèce immunoréactive.

Généralement, le couplage d'un latex fluorescent à une espèce immunoréactive est effectué par l'intermédiaire d'un ou plusieurs groupement(s) fonctionnel(s), réactif(s) et présent(s) à la surface de ces particules.

La procédure classique pour réaliser ce type de couplage, implique la création d'une liaison covalente avec cette espèce immunoréactive, par simple réaction chimique. Les groupements chimiques, présents à la surface des particules, peuvent, soit réagir directement avec les composés immunoréactifs porteurs de fonctions amine ou sulfhydryle libres ou, indirectement après activation. A titre de groupements fonctionnels , on peut en particulier citer les groupements carboxyle, halogénoalkyle, alkylsulfonyle et vinylsulfonyle. Dans le cas de groupements aldéhydiques et époxy, ces derniers peuvent être activés chimiquement au préalable pour conduire à une fonction présentant une activité supérieure vis à vis des espèces immunoréactives.

Sous le terme espèce immunoréactive, on désigne tout composé chimique ou biologique porteur d'au moins un site susceptible de se complexer à une autre molécule spécifique dite récepteur. A titre d'exemples d'espèces immunoréactives, on peut citer les amines primaires, les acides aminés, les peptides, les protéines, les lipoprotéines ou des microrganismes de type virus ou bactéries par exemple. Il peut également s'agir d'un anticorps ou d'une enzyme. Cette espèce immunoréactive a pour mission essentielle de réagir avec une autre espèce, soit par simple affinité biologique, soit par réaction chimique, en se complexant par l'intermédiaire d'une de ses fonctions à un site récepteur de l'espèce à doser.

L'addition de ces espèces immunoréactives, aux particules de latex fluorescentes, se réalise selon des procédés classiques, en utilisant des réactions connues qui ne seront pas rappelées ici.

Les latex fluorescents selon l'invention sont plus particulièrement destinés à intervenir directement ou indirectement dans des analyses biologiques. Ils peuvent par exemple être employés à titre de réactifs dans les tests immunologiques, de scintillateurs, de standards de calibration en cytofluorométrie en flux par exemple ou encore comme marqueurs cellulaires. On réalise dans ce dernier cas une phagocytose des particules d'un latex fluorescent par les cellules à étudier.

En ce qui concerne plus particulièrement les dosages immunologiques, ils mettent de préférence en oeuvre des latex fluorescents selon l'invention fonctionnalisés en outre par au moins une espèce immunoréactive.

Un avantage important de la fluorescence sur d'autres techniques comme l'UV-visible ou l'émission radioactive, est de permettre en outre le dosage de composés inhibiteurs. Cette caractéristique offre aux latex fluorescents selon l'invention des applications pratiques supplémentaires telles que les dosages de traces de métaux lourds, de l'oxygène...Cette technique fait déjà l'objet d'applications en immunologie. Il s'agit plus particulièrement de la méthode d'immuno-fluorescence inverse.

Les exemples présentés ci-après permettront de mettre en évidence d'autres avantages et caractéristiques de la présente invention sans toutefois en limiter la portée.

Les latex fluorescents, faisant l'objet des exemples ci-après, ont été caractérisés par leur seuil de détection en émission fluorescente et, leurs particules respectives, par leur taux d'occupation en fluorochromes selon les méthodes suivantes:

## A - Mesure du seuil de détection de fluorescence

Elle consiste à déterminer la concentration minimale en particules détectables par le fluorimètre dans des conditions acceptables sur le plan du rapport signal/bruit (>10), par un fluorimètre conventionnel et sans désoxygénation du milieu. Ce fluorimètre conventionnel comprend une source lumineuse polychromatique (lampe Xénon 150 W), des monochromateurs à réseau, un directeur classique et un dispositif de lecture et/ou d'enregistrement du photocourant induit. Il ne comporte en outre aucun dispositif particulier de traitement du signal. Il est clair que cette concentration minimale détectable dépend fortement des conditions d'analyse.

En conséquence, les valeurs indiquées dans les exemples ci-après, déterminées dans des conditions standards, doivent être considérées comme indicatrices des performances minimales des latex fluorescents analysés.

Pour ce faire, on réalise, à des longueurs d'ondes d'excitation et d'émission précises et pour des paramètres optiques ou électroniques du fluorimètre préalablement définis et fixés, une étude de l'intensité émise en fonction de la dilution du latex de départ.

A titre indicatif, les determinations des seuils de détection présentés ci-après ont été effectués à l'aide des lasers suivants:
* laser Helium-Néon (HeNe): 543, 633 nm (593, 612)
* laser Argon/Argon ionisé: 458, 488 et 515 nm (raies principales)

La valeur de la concentration minimale est ensuite déduite à la lecture de la courbe correspondante d'intensité/concentration.

## B - Détermination du taux d'occupation d'une particule en fluorochromes

Ce taux est accessible par dissolution d'un poids connu de latex sec dans un solvant commun au polymère et au fluorochrome. En général, il s'agit du toluène. La concentration en fluorochrome est alors déterminée par absorption UV-visible. La concentration en particules est déduite par calcul à partir du poids de polymère sec, de la taille des particules et de la masse volumique du polymère.

le taux d'occupation $\eta_p$ est alors donné par le rapport:

$$\eta_p = \frac{\text{concentration en fluorochrome}}{\text{concentration en particules}}$$

## EXEMPLE 1

### - Préparation d'un latex fluorescent à la tétraphénylporphine

Ce latex fluorescent est préparé selon la technique de gonflement en utilisant les réactifs suivants:
Latex Estapor $K_1030$ lot 326® 130 g à 7,75 %,
(latex de polystyrène carboxylé de $\varnothing$ 0,3µ)
Toluène 20 g
Acétone 80 g,
Tétraphénylporphine 80 mg,
Laurate de potassium 0,3 g sec,
$NH_4OH$ à 20 % qs pour pH 10.

### - Mode opératoire :

La tétraphénylporphine est dissoute dans le toluène puis additionnée d'acétone. Le pH du latex est ajusté par $NH_4OH$ à une valeur de 10. Le laurate de potassium est ensuite additionné au latex et l'ensemble est homogénéisé quelques minutes sous agitation.
On ajoute ensuite lentement la solution de fluorochrome au latex. La suspension résultante est agitée pendant 3 heures à 40°C. Les solvants organiques sont ensuites éliminés par distillation le plus lentement posible. Le latex fluorescent ainsi obtenu est lavé par ultrafiltration.

### - Caractéristiques du latex fluorescent :

* diamètre des particules 0,293 µm.
* absence de fluorochrome dans le sérum (mesure de la fluorescence de l'ultrafiltrat).
* le taux moyen d'occupation est de 60 000 molécules de TPP par particule de latex.
  La fluorescence du latex est stable sous irradiation et comparable à celle d'une solution de TPP dans le toluène :
  $\lambda_{ex}$ = 420 nm (intense), 515 nm et 550 nm (faible),
  $\lambda_{em}$ = 656 nm et 720 nm (rapport 720/656 = 0,3).
* Le seuil de détection de fluorescence est de $2.10^{-13}$ moles de particules par litre de latex, ce qui indique une très grande sensibilité de détection.

## EXEMPLE 2

### - Préparaltion d'un latex fluorescent au 5-12bis-phényléthynyl-naphtacène (5-12 BPEN),

Ce latex est obtenu conformément au mode opératoire décrit en exemple 1 en mettant en oeuvre les réactifs suivants:
Latex Estapor $K_1030$ lot 326® 65 g à 7,75 %,
(Latex de polystyrène carboxylé de $\varnothing$ 0,3µ)
5-12 BPEN                40 mg
Acétone                  60 g
Toluène                  10 g
Laurate de potassium     0,3 g sec
$NH_4OH$ à 20 % q.s. pour pH 10. Dans ce cas le gonflement est réalisé au cours de 3 heures à 50°C. Après distillation, le latex est lavé par ultrafiltration et caractérisé.

### Caractéristiques du latex fluorescent

* diamètre des particules: 0,293 µm,
* le taux moyen d'occupation des particules est de 125 000 molécules de fluorochrome par particule de latex.
* la fluorescence est stable sous irradiation excitatrice.

. l'excitation maximale est à 565 nm mais une émission intense peut-être obtenue par excitation au laser He-Ne à 543 nm (80 %) ou au laser Argon ionisé à 515 nm (80 %).

. Emission :

$\qquad$ 568 nm (maximun)

$\qquad$ 610 nm (610/563 = 0.3)

* le seuil de détection est très bas, puisqu'il se situe vers $4.10^{-15}$ moles de particules par litre de latex. La sensibilité du latex fluorescent est donc excellente.

## EXEMPLE 3

### - Préparation d'un latex fluorescent au 5-12bis-phényléthynyl-naphtacène (5-12 BPEN)

Ce latex est obtenu conformément au mode opératoire décrit en exemple 1 en mettant en oeuvre les réactifs suivants:

Latex Estapor $K_1$010 lot 786® 79,7 g à 12,54 %,

(Latex de polystyrène carboxylé de $\varnothing$ 0,1 $\mu$)

5-12 BPEN    78 mg

Acétone      222 g

Toluène      14,5 g

Le fluorochrome est dissous dans 122g d'acétone et 14,5g de toluène. L'ensemble est laissé sous agitation pendant 12 heures. En parallèle, on incorpore 100g d'acétone au latex. Les deux mélanges sont ensuite réunis. On procède au gonflement à 50°C pendant 3 heures. Après distillation, le latex est lavé par ultrafiltration et caractérisé.

### *Caractéristiques du latex fluorescent*

* diamètre des particules : 0,185 $\mu$m,
* le taux moyen d'occupation des particules est de 30 000 molécules de fluorochrome par particule de latex.
* la fluorescence du latex est intense:

$\lambda_{ex}$ = 515 nm, 555 nm,

$\lambda_{em}$ = 568 nm, 610 nm.

* le seuil de détection se situe vers $2.10^{-14}$ moles de particules par litre de latex.

## Exemple 4

### - Préparation d'un latex fluorescent au 9 - 10 bis-phényléthynyl-anthracène (9-10 BPEA)

Ce latex est obtenu conformément au mode opératoire décrit en exemple 1 en mettant en oeuvre les réactifs suivants :

Latex Estapor $K_1$080 lot 705® 17g à 29,4 %

Latex de polystyrène carboxylé de $\varnothing$ 0.9 $\mu$m

9-10 BPEA      22 mg

Acétone        49 g

Cyclohexanone  5 g

Dans ce cas le gonflement est réalisé au cours de 3 heures à 50°C. Après distillation, le latex est lavé par ultrafiltration et caractérisé.

### *Caractéristiques du latex fluorescent :*

* diamètre des particules: 0,899 $\mu$m
* le taux moyen d'occupation des particules est de $2.910^6$ molécules de fluorochrome par particule de latex.
* la fluorescence du latex est intense :

$\lambda$ex = 440 nm, 455 nm, 468 nm (intense)

$\lambda$em = 480 nm (intense), 510 nm

* le seuil de détection est très faible, puisqu'il se situe vers $7.10^{-17}$ moles de particules par litre de latex.

La sensibilité du latex fluorescent est donc excellente.

**EXEMPLE 5**

- Préparation d'un latex fluorescent au 1,8 dichloro 9-10 bis-phényléthynyl-anthracène (1,8 $Cl_2$-9-10 BPEA)

Ce latex est obtenu conformément au mode opératoire décrit en exemple 1 en mettant en oeuvre les réactifs suivants:

Latex Estapor $K_1$080 lot 326® 65g à 8,13 %
Latex de polystyrène carboxylé de $\varnothing$ 0,3 μm

| | |
|---|---|
| 1,8 $Cl_2$ 9-10 BPEA | 55 mg |
| Acétone | 98 g |
| Toluène | 7,5 |
| Laurate de potassium | 0,05 g |

$NH_4OH$ à 20 % q.s. pour pH10.
Dans ce cas le gonflement est réalisé au cours de 3 heures à 50°C. Après distillation, le latex est lavé par ultrafiltration et caractérisé.

*Caractéristiques du latex fluorescent :*

* diamètre des particules: 0,328μm
* le taux moyen d'occupation des particules est de 276.000 molécules de fluorochrome par particule de latex.
* la fluorescence du latex est intense :
  $\lambda$ex = $\underline{470}$ nm (principal) , 490 nm
  $\lambda$em = $\underline{525}$ nm (principal), 560 nm
* le seuil de détection est très faible, puisqu'il se situe vers $1,5.10^{-15}$ particules par litre de latex. La sensibilité du latex fluorescent est donc excellente.

**Revendications**

1 - Latex fluorescent dont les particules contiennent au moins un fluorochrome hydrophobe encapsulé caractérisé en ce qu'il possède un seuil de détection de fluorescence inférieur ou égal à $10^{-12}$ moles de particules par litre de latex et en ce que ledit fluorochrome hydrophobe et encapsulé au sein des particules du latex est un polyaromatique condensé, un de ses dérivés substitués, la tétraphénylporphine eVou un des complexes organométalliques de celle-ci.

2- Latex fluorescent, selon la revendication 1, caractérisé en ce que le fluorochrome est de préférence choisi parmi les 9,10 diphényl-anthracènes (9,10 DPA), 9-10 bis-phényl-éthynyl-anthracènes (9-10 BPEA), 1,8 dichloro 9-10 bis-phényl-éthynyl-anthracènes (1,8 $Cl_2$-9-10.BPEA) les 5-12 bis-phényl-éthynyl-naphtacènes (5-12 BPEN), 6,13-bis (phényléthynyl)pentacènes, tétrabenzo(de,hi,op,st)pentacènes et leurs dérivés substitués, la tétraphénylporphine et la tétraphénylporphine de zinc.

3 - Latex fluorescent selon l'une quelconque des revendications précédentes caractérisé en ce que la taille de ses particules est comprise entre 0,01 microns et 20 microns.

4 - Latex fluorescent selon l'une quelconque des revendications précédentes caractérisées en ce que les particules constituent entre 0,2 et 50 % de son poids.

5 - Latex fluorescent, selon l'une quelconque des revendications précédentes caractérisé en ce que son seuil de détection est compris entre $10^{-12}$ et $10^{-18}$ moles de particules par litre de latex.

6 - Latex fluorescent, selon l'une quelconque des revendications précédentes, caractérisé en ce que le polymère constituant ses particules est un homopolymère ou copolymère contenant des motifs dérivés de monomères vinylaromatiques, éthyléniques, d'acides ou d' esters alcanoïques ou éthylèniques éventuellement fonctionnalisés.

7 - Latex fluorescent, selon l'une quelconque des revendications précédentes caractérisé en ce qu'il est en outre fonctionnalisé par au moins une espèce immunoréactive.

8 - Procédé de préparation d'un latex selon l'une des revendications 1 à 7 mettant en oeuvre une étape choisie parmi:
- l'introduction, en cours de polymérisation du ou des monomère(s) constituant les particules de latex, d'un fluorochrome en suspension dans une fraction du ou d'un des monomère(s) et

- le mélange d'un fluorochrome hydrophobe et solubilisé dans un solvant non aqueux avec une dispersion aqueuse de particules de latex, caractérisé en ce que ledit fluorochrome mis en oeuvre est choisi parmi un polyaromatique condensé, un de ses dérivés substitués, la tétraphénylporphine ou un complexe organométallique de celle-ci et en ce que l'on récupère un latex possèdant un seuil de détection de fluorescence inférieur à $10^{-12}$ moles de particules par litre de latex.

9 - Application d'un latex fluorescent selon l'une des revendications 1 à 7 à titre de réactif dans les tests immunologiques, en immuno-fluorescence inverse, de scintillateurs, de standards de calibration en cytofluorométrie en flux et/ou de marqueurs cellulaires.

Office européen
des brevets

**RAPPORT DE RECHERCHE EUROPEENNE**

Numero de la demande

EP    93 40 0851

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 308 233 (EASTMAN KODAK CO) * page 7, colonne 11, ligne 24 - ligne 49 * --- | 1-9 | C08J3/20 G01N33/58 |
| A | EP-A-0 201 211 (WHITTAKER CORPORATION) --- | | |
| A | FR-A-2 159 043 (ELECTROPRINT, INC.) --- | | |
| A | EP-A-0 305 536 (NIPPON KAYAKU K . K.) --- | | |
| A | US-A-4 199 363 (T. J. CHEN) ----- | | |

**DOMAINES TECHNIQUES RECHERCHES (Int. Cl.5)**

G01N
C08J

Le présent rapport a été établi pour toutes les revendications

| Lieu de la recherche | Date d'achèvement de la recherche | Examinateur |
|---|---|---|
| LA HAYE | 23 JUIN 1993 | CARTAGENA   ABELLA P |